# EUROPEAN PATENT APPLICATION

(11) **EP 4 800 095 A1**
(43) Date of publication of application: **02.09.2026**
(21) Application number: 24882835.2
(22) Date of filing: 24.10.2024
(51) Int. Cl.: C12M 3/00, C12M 3/06, C12M 1/00, C12N 5/077, C12N 5/071

(54) **BONE TISSUE CHIP COMPRISING BONE MATRIX MIMIC AND FABRICATION METHOD THEREFOR**

(30) Priority: 27.10.2023 KR 20230145229
(71) Applicant: Dongguk University Wise Campus Industry-Academy Cooperation Foundation, Gyeongju-si, Gyeongsangbuk-do 38066 (KR); Industry Academic Cooperation Foundation of Yeungnam University, Gyeongsan-si, Gyeongsangbuk-do 38541 (KR)
(72) Inventor: KWAK, Bong Seop, Goyang-si Gyeonggi-do 10324 (KR); LIM, Ji Seok, Gyeongsan-si Gyeongsangbuk-do 38603 (KR)
(74) Representative: Mewburn Ellis LLP
(86) International application number: PCT/KR2024/016230
(87) International publication number: WO 2025/089808

(57) **Abstract**

The present invention relates to a bone tissue chip comprising a bone matrix mimic and a capillary mimic to study bone diseases *in vitro,* and a fabrication method therefor. The bone matrix mimic fabricated according to the present invention can simulate the microenvironments of a healthy bone matrix and bone disease *in vitro,* and the bone tissue chip comprising the bone matrix mimic comprises a first channel portion having a bone microenvironment and a second channel portion having a capillary-like environment, which are in communication with each other through micropores packaged with a monocellular membrane, thus can simulate interactions between a bone matrix and surrounding capillaries of the body on a chip horizontally, and therefore the present invention is provided as a bone tissue chip that can be used to develop therapeutic agents for special bone diseases such as cancer metastasis indicated by bone density.

## Description

### Technical Field

The present disclosure relates to a bone tissue chip including a bone matrix mimic and a capillary mimic to study bone diseases *in vitro*, and a method of fabricating the same.

### Background Art

As the aging population increases, the number of patients with bone diseases is rapidly increasing. Osteopenia and osteoporosis, which are caused by decreased bone density, have been classified as geriatric diseases, but recently, with the rapid aging of the population, the number of patients suffering from bone diseases such as osteoporosis before the age of 50 is increasing. Additionally, as bone density decreases, the risk of developing secondary diseases such as cancer metastasis or infectious bone diseases increases, and thus, there is a need for the development of means to facilitate the discovery of specific and effective therapeutic agents for these diseases.

Many researchers have recently been actively conducting design optimization research for specific purposes using microfluidic technology. This technology may provide quantitative information for drug screening and drug delivery systems by implementing the properties and functions of different organs on micro-chips and mimicking the interactions between cells and the surrounding microenvironment. Therefore, biomimetic organ-on-a-chip based on microfluidics systems is a major alternative that is capable of overcoming the limitations of animal testing, such as cost, time, ethical issues, and human suitability, and is a platform with the potential to be usefully applied in the preclinical process of new drug development.

### [Prior-Art Documents]

### [Patent Documents]

Korean Patent Registration No. 10-1898093 (published on September 12, 2018)
Korean Patent Registration No. 10-1959208 (published on March 12, 2019)
Korean Patent Application Publication No. 10-2023-0048741 (published on April 12, 2023)

### Disclosure of the Invention

### Technical Goals

An object of the present disclosure is to provide a bone matrix mimic implemented with the characteristics of *in vivo* bone matrix observed in bone diseases on a chip, in order to study bone diseases *in vitro*.

### Technical Solutions

The present disclosure provides a bone tissue chip including: a first channel portion in which osteocytes are cultured; and a second channel portion arranged side-by-side in the same direction as the first channel portion and in which vascular endothelial cells are cultured, wherein one side of the first channel portion and one side of the second channel portion communicate with each other through one or more micropores.

In addition, the present disclosure provides a method of fabricating a bone matrix mimic on the bone tissue chip, including: (1) preparing a fibrin gel including a first culture medium, osteocytes, a thrombin solution, and a fibrinogen solution, and including at least one of osteoblasts and osteoclasts; (2) injecting the fibrin gel into the first channel portion and culturing the same to induce cross-linking of the osteocytes; (3) injecting a fibronectin solution into the second channel portion to coat a wall surface of the second channel portion with the fibronectin; and (4) injecting vascular endothelial cells cultured in a second culture medium into the second channel portion and culturing the same to form a monocellular membrane of the vascular endothelial cells covering the micropores.

In addition, the present disclosure provides a bone matrix mimic fabricated by the above-described method.

### Advantageous Effects

A bone matrix mimic fabricated according to the present disclosure can replicate microenvironments of a healthy bone matrix and a bone disease *in vitro*, and a bone tissue chip including the bone matrix mimic includes a first channel portion where a bone microenvironment is formed and a second channel portion where a capillary-like environment is formed, which are in communication with each other through micropores wrapped with a monocellular membrane, so that interaction between the *in vivo* bone matrix and surrounding capillaries can be implemented horizontally on a chip, and thus it may be provided as a bone tissue chip that can be utilized for developing therapeutic agents for a special bone disease such as cancer metastasis associated with bone density.

### Brief Description of Drawings

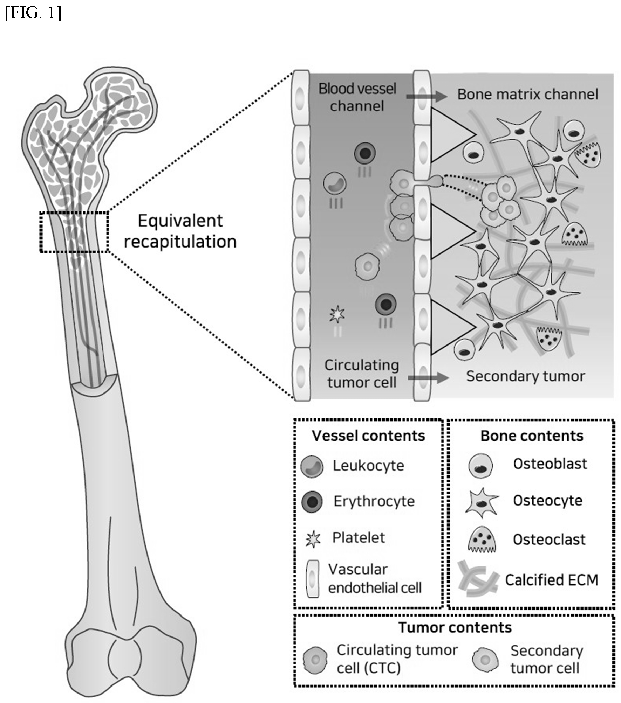

FIG. 1 is a diagram illustrating a bone tissue environment to be mimicked in the present disclosure.
FIG. 2 shows microscopic images illustrating steps of encapsulating osteoblasts within droplets via a droplet-based microfluidics system to differentiate osteoblasts into osteocytes according to the present disclosure, followed by preparing and collecting osteoblast spheroids (OB spheroids) for subsequent differentiation into osteocytes.
FIG. 3 shows the results of analyzing the expression of bone sialoprotein-2 (BSP-2), which is an osteoblast-specific marker, and dentin matrix protein 1 (DMP-1), which is an osteocyte-specific marker, to evaluate whether osteoblast spheroids (OB spheroids) have differentiated into osteocytes according to the present disclosure.
FIG. 4 is a micrograph of osteoclasts differentiated from macrophages according to the present disclosure.
FIG. 5 is a micrograph of a cross-section of a bone tissue chip in which a bone matrix mimic is formed according to the present disclosure.
FIG. 6 is a photograph of an organ-on-a-chip in which a bone tissue chip is fabricated according to an aspect of the present disclosure.
FIG. 7 is a micrograph of a bone-microenvironment channel of a bone tissue chip fabricated according to the present disclosure.
FIG. 8 shows the results of analyzing the level of calcium deposition to evaluate a bone matrix mimic fabricated on a bone tissue chip according to the present disclosure.
FIG. 9 shows the results of analyzing the calcium deposition levels of bone matrix mimics that mimic osteopenia and osteoporosis, which are bone diseases, on a bone tissue chip according to the present disclosure.
FIG. 10 shows the results of evaluating changes in calcium deposition levels according to the concentration of cultured cells on a 96-well plate (off-chip).
FIG. 11 shows the results of evaluating changes in calcium deposition levels according to the concentration of cultured cells on a bone tissue chip.
FIG. 12 shows micrographs showing a fabricating process of a bone matrix mimic that mimics bone metastatic cancer, a bone disease, on a bone tissue chip according to the present disclosure.

### Best Mode for Carrying Out the Invention

Terms used in the present specification are selected from the most widely used general terms possible while taking into account functions of the present disclosure, but these may vary depending on the intention of a person skilled in the art, precedents, the emergence of new technologies, and the like. Additionally, in certain cases, some terms are arbitrarily selected by the applicant, and in such cases, the detailed meanings thereof will be described in relevant parts of the description of the invention. Therefore, the terms used in the present disclosure should not be simply defined as names of terms, but should be defined based on the meaning of the terms and the overall content of the present disclosure.

Unless otherwise defined, all terms used herein, including technical or scientific terms, have the same meaning as commonly understood by a person skilled in the art to which the present disclosure pertains. Terms defined in commonly used dictionaries should be interpreted to have a meaning consistent with their meaning in the context of the relevant technology, and will not be interpreted in an idealized or overly formal sense unless expressly defined in this application.

Hereinafter, the present disclosure will be described in detail.

The present disclosure provides a bone tissue chip including a first channel portion in which osteocytes are cultured and a second channel portion arranged side-by-side in the same direction as the first channel portion and in which vascular endothelial cells are cultured, wherein one side of the first channel portion and one side of the second channel portion communicate with each other through one or more micropores.

The first channel portion is designated as a bone-microenvironment channel, and is filled with a fibrin gel containing a cell culture medium, and is a space in which osteocytes, osteoblasts, and osteoclasts are cultured to form a bone matrix mimic similar to the *in vivo* bone matrix.

The second channel portion is a space in which vascular endothelial cells are cultured to form a capillary mimic, wherein a monocellular membrane of the vascular endothelial cells is formed on a wall surface thereof. Cell culture media, reagents for analysis, platelets, nutrients, infectious agents, or cancer cells may be injected into the second channel portion as needed. A microfluidic system may be applied to the second channel portion to allow for a continuous flow therein. Specifically, an inlet port and an outlet port are installed in the channel, and the inlet and outlet ports may be connected to a pump or a mixer through a tube. The pump and mixer may be used to mix the cell culture media and substances to be analyzed, or their concentrations may be varied over time, and the flow rate of a fluid passing through the channel may be adjusted.

One or more micropores present on one side where the first channel portion and the second channel portion contact each other have a diameter of 100 µm to 200 µm, and are formed at intervals of a specific size created by arranging pillars of a constant shape in a row.

Cells may be attached to a wall surface on one side of the second channel portion where the micropores are present, and the micropores provide passages through which the cell culture media, reagents for analysis, platelets, nutrients, infectious agents, or cancer cells supplied to the second channel portion can migrate into the first channel portion.

In one aspect of the present disclosure, the first channel portion is a channel having a width of 800 µm and a depth of 200 µm, and the second channel portion is a channel having a width of 1,000 µm and a depth of 200 µm. One side where the two channels contact each other is provided with five equilateral triangular pillars, each having a side length of 300 µm, arranged in a row to separate the two channels. One edge of each equilateral triangular pillar is positioned toward the first channel, while the face opposite to said edge is positioned toward the second channel. Micropores are formed by the gaps between the equilateral triangular pillars relative to the second channel side, and the diameter of the micropores is 150 µm.

The bone tissue chip may be fabricated from a polymeric organosilicon compound, specifically, but not limited to, polydimethylsiloxane (PDMS).

In addition, the present disclosure provides a method of fabricating a bone matrix mimic on the bone tissue chip, including steps of: (1) preparing a fibrin gel including a first culture medium, osteocytes, a thrombin solution, and a fibrinogen solution, and including at least one of osteoblasts and osteoclasts; (2) injecting the fibrin gel into the first channel portion and culturing the same to induce cross-linking of the osteocytes; (3) injecting a fibronectin solution into the second channel portion to coat a wall surface of the second channel portion with the fibronectin; and (4) injecting vascular endothelial cells cultured in a second culture medium into the second channel portion and culturing the same to form a monocellular membrane of the vascular endothelial cells covering the micropores.

The osteocytes are differentiated from spheroids derived from the osteoblasts, and specifically, the spheroids may be produced from osteoblasts cultured inside droplets through a droplet-based microfluidics system.

The osteoclasts are differentiated from immune cells, and the immune cells are monocytes or macrophages.

The bone matrix mimic of the present disclosure mimics a healthy normal bone matrix environment or a bone matrix environment of a bone disease.

Specifically, when the bone matrix mimic mimics a healthy normal bone matrix, the fibrin gel contains the osteocytes at a concentration of 1.25 × 10⁷ cells/mL to 2.5 × 10⁷ cells/mL, and the osteoblasts at a concentration of 1.25 × 10⁶ cells/mL to 5 × 10⁶ cells/mL, wherein a ratio of the osteocytes to the osteoblasts is 10:2.

In addition, when the bone matrix mimic mimics a bone matrix of osteopenia among bone diseases, the fibrin gel contains the osteocytes at a concentration of 1.25 × 10⁷ cells/mL to 2.5 × 10⁷ cells/mL, the osteoblasts at a concentration of 1.25 × 10⁶ cells/mL to 2.5 × 10⁶ cells/mL, and the osteoclasts at a concentration of 1.25 × 10⁶ cells/mL to 2.5 × 10⁶ cells/mL, wherein a ratio of the osteocytes, the osteoblasts, and the osteoclasts is 10:1:1.

In addition, when the bone matrix mimic mimics a bone matrix of osteoporosis among bone diseases, the fibrin gel contains the osteocytes at a concentration of 1.25 × 10⁷ cells/mL to 2.5 × 10⁷ cells/mL, and the osteoclasts at a concentration of 1.25 × 10⁶ cells/mL to 5.0 × 10⁶ cells/mL, wherein a ratio of the osteocytes to the osteoclasts is 10:2.

Further, when the bone matrix mimic mimics a bone matrix of bone metastatic cancer among bone diseases, the method further includes steps of, after step (5): (6) injecting cancer cells cultured in a third culture medium into the second channel portion at a flow rate of 1.0 µL/min to 2.0 µL/min; and (7) supplying a mixed culture medium including the first culture medium, the second culture medium, and the third culture medium into the second channel portion to perform co-culturing, when the cancer cells are attached to the monocellular membrane of the vascular endothelial cells covering the micropores.

The first culture medium is α-MEM (Minimum Essential Medium) for culturing bone cells, the second culture medium is EBM-2 (Endothelial Basal Medium-2) for culturing vascular endothelial cells, and the third culture medium is DMEM/F12 (Dulbecco's Modified Eagle Medium /F12) for culturing cancer cells.

The vascular endothelial cells are human umbilical vein endothelial cells (HUVECs).

In addition, the present disclosure provides a bone matrix mimic fabricated by the above-described method.

### Modes for Carrying Out the Invention

Hereinafter, the present disclosure will be described in detail with reference to examples to facilitate understanding of the present disclosure. The following examples are merely illustrative of the content of the present disclosure, and the scope of the present disclosure is not limited to the following examples. The examples of the present disclosure are provided to more completely explain the present disclosure to a person skilled in the art.

### Example 1. Cell preparation and differentiation

To fabricate a bone matrix mimic of the present disclosure, osteocytes, osteoblasts, and osteoclasts were prepared. MC3T3-E1 cells, an osteoblast cell line, were used and cultured in α-MEM (Minimum Essential Medium) containing 10% FBS (Fetal Bovine Serum), 100 µg/mL streptomycin, and 100 U/mL penicillin under conditions of 37°C and 5% CO₂.

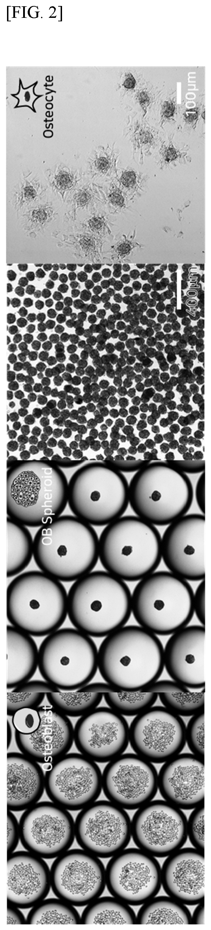

Osteocytes were prepared by differentiating MC3T3-E1 cells. To differentiate MC3T3-E1, the osteoblast cell line, into osteocytes, as shown in FIG. 2, the osteoblasts were prepared into osteoblast spheroids (OB spheroids) and then differentiated into osteocytes. To prepare spheroid-shaped osteoblasts (OB spheroids), MC3T3-E1 cells were encapsulated within droplets using a droplet-based microfluidics system. The droplet-based microfluidics system used a commercially available fluid chip or a separately manufactured fluid chip. An α-MEM (Minimum Essential Medium) culture medium containing MC3T3-E1 cells at a concentration of 5 × 10⁶ cells/ml was injected into the aqueous phase inlet of the droplet-based microfluidics system, and an oil phase solution containing 5% (v/v) surfactant (Pico-Surf^{TM}, Dolomite Microfluidics, UK) and fluorinated oil (Novec^{TM} 7500, 3M, MN) was injected into the oil phase inlet of the droplet-based microfluidics system to form droplets in which the MC3T3-E1 cells were uniformly encapsulated. The injection flow rates of the culture medium and the oil phase solution were 40 µl/min and 120 µl/min, respectively, and the droplets formed at the microchannel cross-section of the droplet-based microfluidics system were collected from an outlet. The collected droplets were cultured for 48 hours at 37°C and 5% CO₂ conditions to prepare osteoblast spheroids (OB spheroids). Whether the osteoblasts within the droplets underwent morphological changes into spheroids was continuously monitored using an optical microscope-equipped live-cell imaging system (Live Cell Instrument, Korea). Live images were captured every 10 minutes for 24 hours. For immune-fluorescence assay, fluorescence images of the spheroid model were analyzed using a fluorescence microscope (Olympus, Japan) coupled with a deconvolution system (Microvolution, CA). By measuring the diameter or perimeter of the spheroid model, circularity, convexity, and elongation were calculated through live image-based measurements using ImageJ software (NIH, Bethesda, MD).

Upon confirming the spheroid shape on the droplets, the droplets were disrupted by treatment with 1H,1H,2H,2H-perfluoro-1-octanol (Sigma-Aldrich, MO) to collect the osteoblast spheroids (OB spheroids). The collected OB spheroids were transferred to a 2D culture dish and cultured for 48 hours at 37°C and 5% CO₂ in α-MEM (Minimum Essential Medium) containing 10% FBS (Fetal Bovine Serum), 100 µg/mL streptomycin, and 100 U/mL penicillin. To evaluate whether the OB spheroids differentiated into osteocytes, expression of BSP-2 (bone sialoprotein-2), an osteoblast-specific marker, and DMP-1 (Dentin matrix protein 1), an osteocyte-specific marker, was analyzed.

The expression of BSP-2 and DMP-1 was confirmed through immune-fluorescence assay. Cells and spheroids were fixed for 60 minutes using a 4% (v/v) paraformaldehyde (Sigma-Aldrich, MO) solution. Blocking and permeabilization were performed using a solution containing 2% (v/v) BSA (bovine serum albumin) and 0.1% (v/v) Triton X-100 (Sigma-Aldrich). The samples were treated with primary antibodies, anti-BSP-2 (1:100, BS-2668R, Bioss Antibodies Inc., Woburn, MA) and anti-DMP-1 (1:100, H00001758-M01, Abnova, Walnut, CA), and allowed to react overnight at 4°C. Subsequently, secondary antibodies for BSP-2 (1:200, ab150077, Abcam, Cambridge, MA) and DMP-1 (1:100, ab150118, Abcam, Cambridge, MA) were applied and reacted for 4 hours at room temperature. For nuclear staining, DAPI (1:500, Hoechst 33342, Invitrogen, Waltham, MA) was applied for 10 minutes at room temperature. BSP-2 was observed at FITC wavelengths (EX: 460-500 nm, EM: 512-542 nm), DMP-1 at RHOD wavelengths (EX: 541-551 nm, EM: 565-605 nm), and cell nuclei at DAPI wavelengths (EX: 325-375 nm, EM: 435-485 nm).

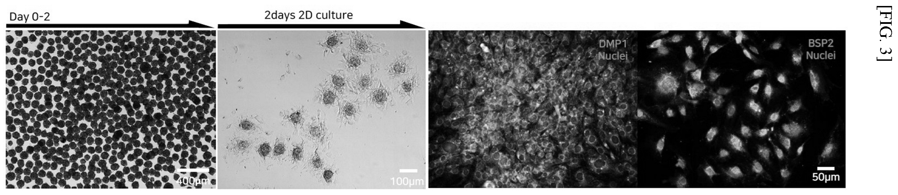

As shown in FIG. 3, as a result of differentiating the osteoblast spheroids (OB spheroids), the cells were cultured into the morphology of osteocytes from the spheroid form, and DMP-1 (Dentin matrix protein 1) (red), an osteocyte-specific marker, was identified via microscopy.

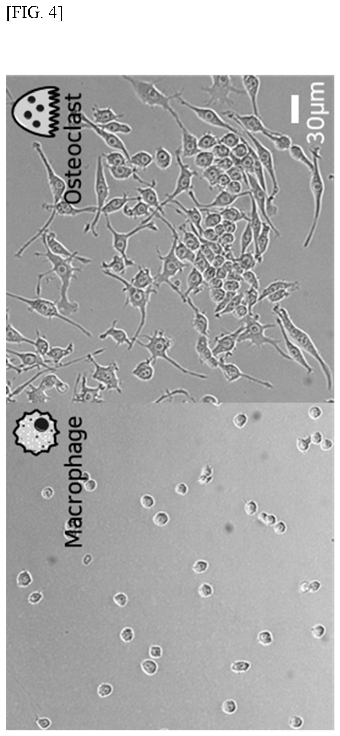

Osteoclasts were prepared by differentiation from RAW 264.7 cells, which is a macrophage cell line. RAW 264.7 cells were cultured in DMEM-HG (Dulbecco's Modified Eagle's Medium - high glucose) (10% FBS, 100 µg/mL streptomycin, 100 U/mL penicillin) at 37°C and 5% CO₂ conditions. The cultured RAW 264.7 cells were seeded in a 6-well plate at a concentration of 1×10⁴ cells/well and differentiated into osteoclasts by culturing for 4 days in DMEM-HG (Dulbecco's Modified Eagle's Medium - high glucose) containing 50 ng/mL of RANKL (Receptor activator of nuclear factor κB ligand) and 35 ng/mL of M-CSF (Macrophage-colony stimulating factor). As shown in FIG. 4, it was confirmed that the spherical RAW 264.7 cells differentiated into irregular-shaped osteoclasts.\

### Example 2. Composition of bone tissue chip

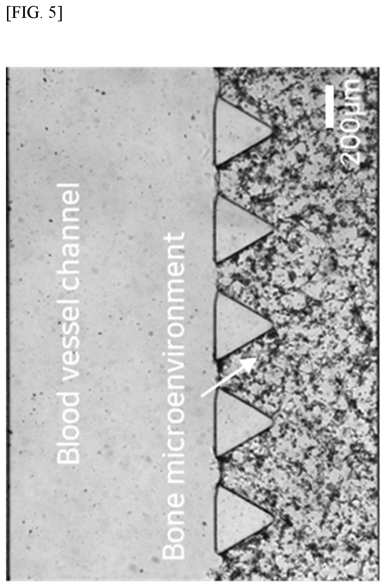

Using the cells prepared in Example 1, a bone tissue chip including a bone matrix mimic for studying bone diseases *in vitro* was fabricated. As shown in FIG. 5, the bone tissue chip of the present disclosure consists of a first channel portion, which is a bone-microenvironment channel forming a bone matrix mimic, and a second channel portion, which is a blood vessel channel forming a capillary mimic.

The bone-microenvironment channel and the blood vessel channel are arranged side-by-side in the same direction. One side of each of the two channels communicates through one or more micropores, and the micropores are formed at intervals of a specific size created by an arrangement of pillars having a uniform shape.

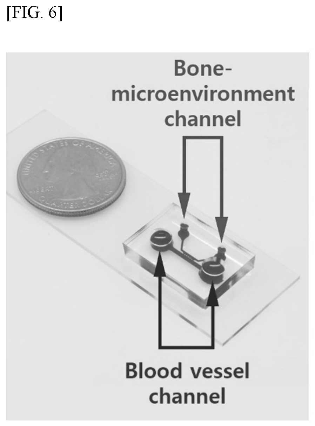

An embodiment of the bone tissue chip of the present disclosure is shown in FIG. 6. In the bone tissue chip, the width of the bone-microenvironment channel is 800 µm, the width of the blood vessel channel is 1,000 µm, and the depth of both channels is 200 µm. Five equilateral triangular pillars, each having a side length of 300 µm, are arranged in a row between the two channels to separate them. One vertex of each equilateral triangular pillar is positioned toward the bone-microenvironment channel, and the side opposite to the vertex is naturally positioned toward the blood vessel channel. Based on the side of the blood vessel channel, micropores are formed by the intervals between the five equilateral triangular pillars, and the diameter of the micropores is 150 µm.

The bone tissue chip was fabricated by replica molding of polydimethylsiloxane (PDMS, Dow Corning, Corning, NY, USA) and formed on a patterned SU-8 silicon wafer. The PDMS replica was punched to create inlets and outlets, and then bonded onto a glass substrate using an oxygen plasma treater (Electro-Technic Products, Chicago, IL, USA).

A bone matrix mimic was fabricated on the bone tissue chip. The cells prepared in Example 1 were suspended in 5 µL of α-MEM (Minimum Essential Medium) culture medium, and then 1 µL of thrombin solution (10 units/mL) and 4 µL of fibrinogen solution (10 mg/mL) were added to prepare an osteo-cell-laden fibrin gel. The fibrin gel was injected into the bone-microenvironment channel such that the final cell concentration was 3.0×10⁷ cells/mL (fibrin gel).

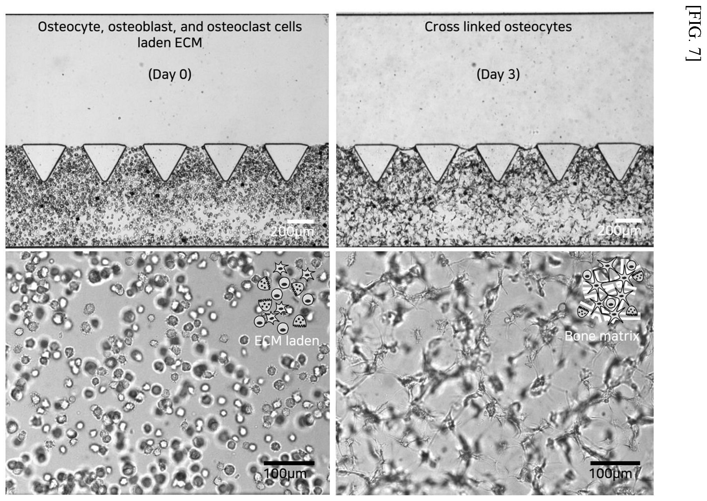

As shown in FIG. 7, an extracellular matrix (ECM) containing osteoblasts, osteocytes, or osteoclasts was finally formed in the bone-microenvironment channel. As a result of culturing the bone tissue chip for 3 days at 37°C and 5% CO₂ conditions, it was microscopically confirmed that the osteocytes cross-linked to form a bone matrix mimic.

### Example 3. Analysis of calcium deposition

To evaluate whether the bone matrix mimic prepared in Example 2 realizes the characteristics of *in vivo* bone matrix, the level of calcium deposition was analyzed. Since bone is a mineralized connective tissue in which calcium serves as a primary component providing bone strength and structure, calcium deposition is a representative method for measuring bone mineralization. Further, calcein is primarily used to identify newly mineralized matrix, and generally, the presence of calcium deposition represents bone strength.

α-MEM culture medium containing 25 µg/ml of calcein (C0875, Sigma-Aldrich, MO) was injected into the bone-microenvironment channel, and the bone-on-a-chip was cultured and stained for 3 days at 37°C and 5% CO₂ conditions. Subsequently, the chip was washed three times with DPBS (Dulbecco's Phosphate-Buffered Saline) for 30 minutes and fixed with a 4% paraformaldehyde solution. After imaging the bone-microenvironment channel using a confocal microscope, the fluorescence intensity of the captured images was measured via the 'IntDen' (Integrated Density) of ImageJ to analyze the calcium level of the fabricated bone matrix mimic.

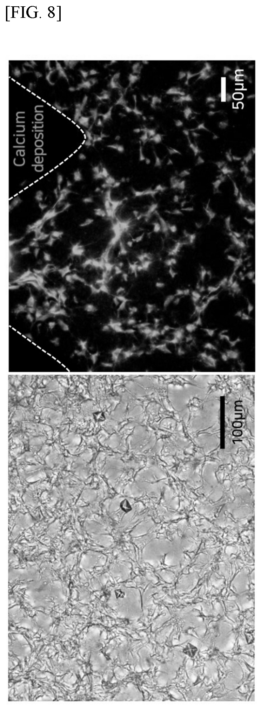

As shown in FIG. 8, it was confirmed that green-stained calcium was deposited in the bone-microenvironment channel of the bone tissue chip fabricated according to the present disclosure. This demonstrates that a bone matrix mimic, similar to the *in vivo* bone matrix, was successfully formed within the bone-microenvironment channel.

### Example 4. Preparation of bone disease models

Using the bone matrix mimic prepared according to the present disclosure, an osteopenia model and an osteoporosis model, which are bone diseases occurring according to bone density, were implemented. For the osteopenia model and the osteoporosis model, bone matrix mimics were prepared in the same manner as in Example 2, except that the ratios of bone cells were mixed differently when preparing the osteo-cell-laden fibrin gel. Table 1 below shows the ratios and cell concentrations of osteocytes, osteoblasts, and osteoclasts in the normal model mimicking healthy bone matrix, and the osteopenia and osteoporosis models mimicking bone diseases. Subsequently, calcium deposition was measured in the same manner as in Example 3 to evaluate whether the characteristics of the bone disease models were successfully implemented.

**TABLE 1**

| | | Osteocyte | Osteoblast | Osteoclast |
|---|---|---|---|---|
| Normal model (Normal) | Ratio | 10 | 2 | 0 |
| | Cell concentration (cells/ml) | 2.5 x 10⁷ | 5.0 x 10⁶ | 0 |
| Osteopenia model (Osteopenia) | Ratio | 10 | 1 | 1 |
| | Cell concentration (cells/ml) | 2.5 x 10⁷ | 2.5 x 10⁶ | 2.5 x 10⁶ |
| Osteoporosis model (Osteoporosis) | Ratio | 10 | 0 | 2 |
| | Cell concentration (cells/ml) | 2.5 x 10⁷ | | 5.0 x 10⁶ |

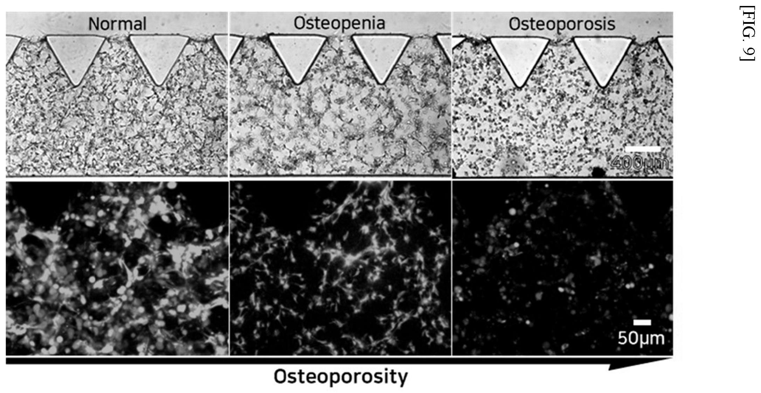

As shown in FIG. 9, the degree of calcium deposition was reduced in the osteopenia and osteoporosis models compared to the normal model, thereby confirming the formation of environments similar to bone diseases characterized by low bone density. This demonstrates that a bone matrix environment similar to bone diseases, such as osteopenia and osteoporosis, was successfully implemented in the bone matrix mimic of the present disclosure.

### Example 5. Evaluation of calcium deposition according to cell concentration

To evaluate whether the bone tissue chip fabricated according to the present disclosure has a superior ability to mimic bone diseases compared to conventional plate culture, changes in calcium deposition levels were assessed in a plate culture model and the chip model according to the concentration of cultured cells. An osteo-cells-laden fibrin gel was prepared with a ratio of osteocytes, osteoblasts, and osteoclasts at 10:1:1, similar to the osteopenia model in Example 4. The fibrin gel was prepared to have a final cell concentration of 3.0 × 10⁶ cells/mL (X1), 7.5 × 10⁶ cells/mL (X2.5), 15.0 × 10⁶ cells/mL (X5), 22.5 × 10⁶ cells/mL (X7.5), or 30.0 × 10⁶ cells/mL (X10), and each was injected into a 96-well plate (off-chip) or the bone tissue chip. Then, the 96-well plate (off-chip) and the bone tissue chip were cultured for 3 days under conditions of 37°C and 5% CO₂. Calcium deposition was measured using the same method as in Example 3 above. The change in calcium deposition level was analyzed through the change in fluorescence intensity according to each cell concentration, based on a fluorescence intensity of 100% under the condition of 30.0 × 10⁶ cells/mL (X10).

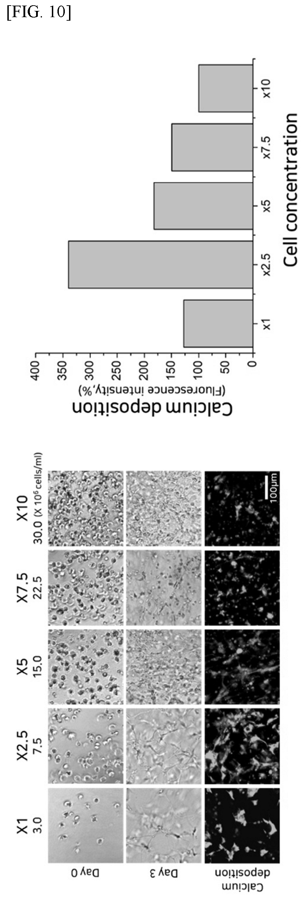

As shown in FIG. 10, the measurement of calcium deposition levels in a 96-well plate (off-chip) resulted in 127.3% at a cell concentration of 3.0 × 10⁶ cells/mL (X1), 339.6% at a cell concentration of 7.5 × 10⁶ cells/mL (X2.5), 181.8% at a cell concentration of 15.0 × 10⁶ cells/mL (X5), and 149.3% at a cell concentration of 22.5 × 10⁶ cells/mL (X7.5). While the highest calcium deposition was observed at the 7.5×10⁶ cells/mL (X2.5) concentration, the levels tended to decrease as the cell concentration increased further, and reproducibility in implementing the bone disease model was found to be poor.

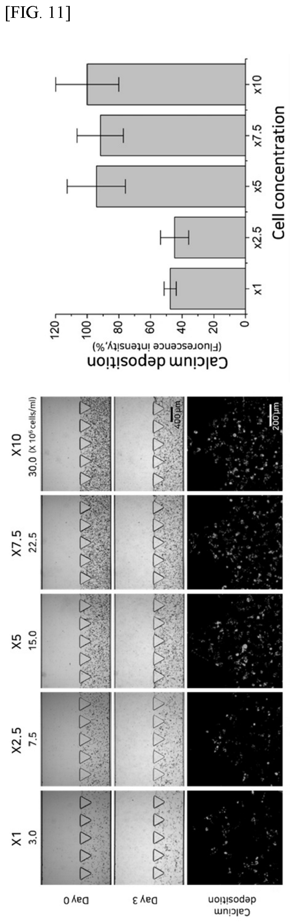

As shown in FIG. 11, the measurement of calcium deposition levels in the bone tissue chip resulted in 47.6±3.8% at a cell concentration of 3.0 × 10⁶ cells/mL (X1), 44.7±8.9% at a cell concentration of 7.5 × 10⁶ cells/mL (X2.5), 94.2±18.4% at a cell concentration of 15.0 × 10⁶ cells/mL (X5), and 91.7±14.5% at a cell concentration of 22.5 × 10⁶ cells/mL (X7.5). Unlike the 96-well plate (off-chip), the bone tissue chip of the present disclosure showed no significant changes in calcium deposition levels even when the number of cells increased from a cell concentration of 15.0 × 10⁶ cells/mL (X5), indicating that calcium deposition reached a saturation point, and demonstrated superior reproducibility for the consistent implementation of a bone disease model.

These results demonstrate that the bone disease model mimicked on the bone tissue chip fabricated according to the present disclosure exhibits less variation in bone density according to cell concentration and superior reproducibility compared to the model replicated on a conventional plate, and that the final cell concentration injected into the bone tissue chip must be at least 15.0×10⁶ cells/mL to achieve excellent implementation of the bone matrix.

### Example 5. Implementation and evaluation of cancer metastasis on bone tissue chip

To evaluate whether a bone metastatic cancer model, which occurs due to cancer metastasis, was successfully mimicked on the bone tissue chip fabricated according to the present disclosure, a bone matrix mimic was formed in the bone-microenvironment channel, and a capillary mimic was formed in the blood vessel channel. Cancer cells were then injected into the blood vessel channel to verify whether cancer metastasis occurred. Following the same method as in Example 2, an osteo-cells-laden fibrin gel was injected into the bone-microenvironment channel and cultured for three days to form the bone matrix mimic.

DMEM-HG (Dulbecco's Modified Eagle's Medium - high glucose) culture medium was injected into the blood vessel channel, and air bubbles present in the channel were removed for 30 minutes at 4°C. Then, the medium in the blood vessel channel was completely removed, and 40 µL of a 0.1 mg/ml fibronectin solution was injected and coated for 30 minutes. After removing the fibronectin solution completely, human umbilical vein endothelial cells (HUVECs) cultured in EBM-2 (Endothelial Basal Medium-2) culture medium were injected into the blood vessel channel at a concentration of 5×10⁶ cells/mL in a volume of 40 µL. The bone tissue chip was tilted 90° so that the bone-microenvironment channel was positioned below, and the HUVEC cells injected into the blood vessel channel were cultured for 40 minutes under conditions of 37°C and 5% CO₂ so that they adhered to the wall separating the two channels. All culture medium in the blood vessel channel was removed, the bone tissue chip was tilted 90° back to its original position, and HUVECs at the same concentration were injected and cultured for 24 hours at 37°C and 5% CO₂ conditions. All culture medium in the blood vessel channel was removed and washed three times for 5 minutes with DPBS (Dulbecco's Phosphate-Buffered Saline). MDA-MB 231 cells, a breast cancer cell line, cultured in DMEM/F12 (Dulbecco's Modified Eagle Medium /F12) medium at a concentration of 5 × 10⁵ cells/ml were injected into the blood vessel channel at a rate of 1.6 µl/min for 3 hours. Afterwards, a mixed culture medium was supplied to the blood vessel channel for culturing cancer cells, and the supplied culture medium was replaced once a day. The mixed culture medium was prepared by mixing equal volumes of α-MEM (Minimum Essential Medium) culture medium for bone cells, EBM-2 (Endothelial Basal Medium-2) culture medium for HUVECs, and DMEM/F12 (Dulbecco's Modified Eagle Medium /F12) culture medium for MDA-MB-231 cells. The locations of HUVECs and MDA-MB-231 cells were detected using a fluorescence microscope after cell membrane staining. HUVECs were stained with PKH67 (Sigma-Aldrich, MO), and MDA-MB-231 with PKH26 (Sigma-Aldrich, MO), and detected at FITC (EX: 460-500 nm, EM: 512-542 nm) and RHOD (EX: 541-551 nm, EM: 565-605 nm) wavelengths, respectively.

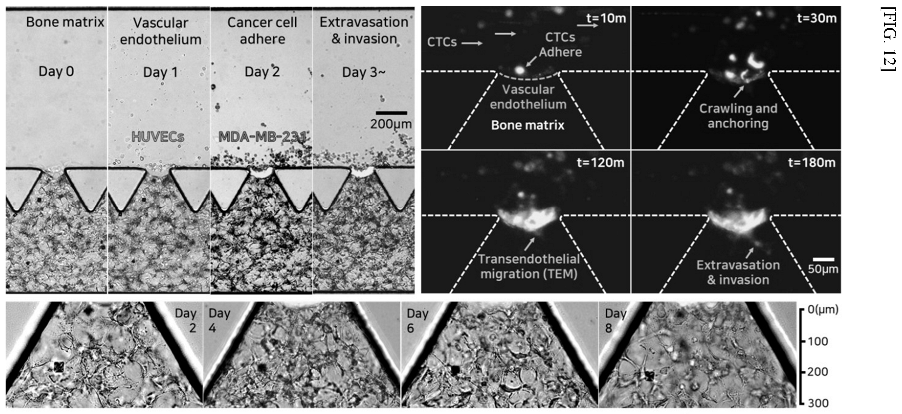

As shown in FIG. 12, it was observed that human umbilical vein endothelial cells (HUVECs) formed a monocellular membrane similar to a blood vessel at the intervals between five equilateral triangular pillars located between the blood vessel channel and the bone-microenvironment channel in the bone tissue chip. In addition, as a result of injecting MDA-MB 231 cells, a breast cancer cell line, into a blood vessel channel, it was confirmed that 10 minutes after the cancer cell injection, the MDA-MB 231 cells adhered to HUVEC cells that formed a monocellular membrane and the initial stage of cancer metastasis, where circulating tumor cells (CTCs) bind to vascular cells for metastasis, was mimicked on the bone-on-a-chip. Approximately 30 minutes after cancer cell injection, the cancer cells exhibited crawling and anchoring behavior, approximately 120 minutes, the cancer cells exhibited transendothelial migration (TEM), and approximately 180 minutes, the cancer cells exhibited infiltration. From the fourth day after cancer cell injection, cancer cells began to invade the bone matrix formed within the bone-microenvironment channel, with deeper invasion observed on days 6 and 8. These results demonstrate that cancer metastasis can be implemented on the bone tissue chip fabricated according to the present disclosure, proving its capability to serve as a bone metastatic cancer model.

As described above, specific parts of the present invention have been described in detail. It is obvious to those skilled in the art that such specific descriptions are merely preferred embodiments and that the scope of the present disclosure is not limited thereby. Therefore, the substantial scope of the present disclosure is defined by the appended claims and their equivalents.

A numerical range includes numerical values defined in the range. Any maximum numerical limit given throughout this specification includes any lower numerical limit as if the lower numerical limit were explicitly written out. Any minimum numerical limit given throughout this specification includes any higher numerical limit as if the higher numerical limit were explicitly written out. Any numerical limitation given throughout this specification will include any desirable numerical range within a broader numerical range, as if the narrower numerical limitation were explicitly written.

## Claims

1. A bone tissue chip comprising:
a first channel portion in which osteocytes are cultured; and
a second channel portion arranged side-by-side in the same direction as the first channel portion and in which vascular endothelial cells are cultured,
wherein one side of the first channel portion and one side of the second channel portion communicate with each other through one or more micropores.

2. The bone tissue chip of claim 1, wherein the micropores have a diameter of 100 µm to 200 µm.

3. A method of fabricating a bone matrix mimic on the bone tissue chip of claim 1, the method comprising:
(1) preparing a fibrin gel comprising a first culture medium, osteocytes, a thrombin solution, and a fibrinogen solution, and comprising at least one of osteoblasts and osteoclasts;
(2) injecting the fibrin gel into the first channel portion and culturing the same to induce cross-linking of the osteocytes;
(3) injecting a fibronectin solution into the second channel portion to coat a wall surface of the second channel portion with the fibronectin; and
(4) injecting vascular endothelial cells cultured in a second culture medium into the second channel portion and culturing the same to form a monocellular membrane of the vascular endothelial cells covering the micropores.

4. The method of claim 3, wherein the osteocytes are differentiated from spheroids derived from the osteoblasts.

5. The method of claim 3, wherein the osteoclasts are differentiated from immune cells.

6. The method of claim 5, wherein the immune cells are monocytes or macrophages.

7. The method of claim 3, wherein the first culture medium is α-MEM (Minimum Essential Medium).

8. The method of claim 3, wherein the fibrin gel comprises the osteocytes at a concentration of 1.25 × 10⁷ cells/mL to 2.5 × 10⁷ cells/mL.

9. The method of claim 3, wherein the fibrin gel comprises osteoblasts at a concentration of 1.25 × 10⁶ cells/mL to 5 × 10⁶ cells/mL.

10. The method of claim 3, wherein the second culture medium is EBM-2 (Endothelial Basal Medium-2).

11. The method of claim 3, wherein the bone matrix mimic mimics a bone disease.

12. The method of claim 11, wherein the bone disease is osteopenia, osteoporosis, or bone metastatic cancer.

13. The method of claim 12, wherein, when the bone disease is osteopenia,
in step (1), the fibrin gel comprises the osteoblasts at a concentration of 1.25 × 10⁶ cells/mL to 2.5 × 10⁶ cells/mL, and comprises the osteoclasts at a concentration of 1.25 × 10⁶ cells/mL to 2.5 × 10⁶ cells/mL.

14. The method of claim 12, wherein, when the bone disease is osteoporosis,
in step (1), the fibrin gel comprises the osteoclasts at a concentration of 1.25 × 10⁶ cells/mL to 5.0 × 10⁶ cells/mL.

15. The method of claim 12, wherein, when the bone disease is bone metastatic cancer, the method further comprises, after step (5):
(6) injecting cancer cells cultured in a third culture medium into the second channel portion at a flow rate of 1.0 µL/min to 2.0 µL/min; and
(7) when the cancer cells are attached to the monocellular membrane of the vascular endothelial cells covering the micropores, supplying a mixed culture medium comprising the first culture medium, the second culture medium, and the third culture medium into the second channel portion to perform co-culturing.

16. The method of claim 3, wherein the vascular endothelial cells are human umbilical vein endothelial cells (HUVECs).

17. A bone matrix mimic fabricated by the method according to any one of claims 3 to 16.
